# EUROPEAN PATENT APPLICATION

(11) **EP 4 039 192 A1**
(43) Date of publication of application: **10.08.2022**
(21) Application number: 21305148.5
(22) Date of filing: 04.02.2021
(51) Int. Cl.: A61B 5/375, A61B 5/377, A61B 5/378, A61B 5/38, A61B 5/381, A61B 5/389, A61B 5/00

(54) **PSYCHOLOGICAL TRAIT DETECTION METHOD BASED ON VIRTUAL REALITY AND NEUROMARKERS AND ASSOCIATED SYSTEM**

(71) Applicant: Open Mind Innovation SAS, 27150 Hébécourt (FR)
(72) Inventor: Chatel-Goldman, Jonas, 34570 Pignan (FR); Kibleur, Astrid, 14000 Caen (FR)
(74) Representative: Cabinet Camus Lebkiri

(57) **Abstract**

The invention relates to a method carried out by a computer for detecting a psychological trait of a user, the method comprising at least the steps of:
- Providing to the user a predetermined sequence of virtual environments, each virtual environment of the sequence of virtual environments being related to a predetermined valence and having a predetermined duration,
- Providing, during the sequence of virtual environments, a passive oddball paradigm to the user comprising providing standard sensory stimulation for a predetermined duration and deviant sensory stimulation for a predetermined duration,
- Acquiring, during the sequence of virtual environments, at least one electroencephalography signal,
- Computing an Event-Related Potential waveform, for each predetermined valence and for each standard and deviant sensory simulation of the oddball paradigm, based on the electroencephalography signal,
- For each valence, computing mismatch negativity based on a difference between the computed Event Related Potentials during deviant sensory stimulation and Event Related Potentials during standard sensory stimulation,
- For each valence, computing a peak around 300 ms post trigger based on the difference between the computed Event Related Potentials during deviant sensory stimulation and Event Related Potentials during standard sensory stimulation,
- Detecting a psychological trait based on the computed mismatch negativity for each valence, on the peak around 300ms for each valence and on predetermined thresholds.

## Description

### TECHNICAL FIELD

The technical field of the invention is the field of neurosciences.

The present document concerns a psychological traits detection method based on virtual reality and neuromarkers and in particular a method for the detection of stress, anxiety and depression by combining virtual reality, electroencephalography and a passive oddball protocol.

### STATE OF THE ART

According to the World Health Organization, mental disorders now affect one in four people, making improving mental health and wellbeing at large scale one of the most important challenges the society is facing today.

Given the size of the problem, it is of paramount importance to tackle it from multiples angles simultaneously. Indeed, researchers and clinicians have identified several difficulties arising from the traditional symptom-based categorical diagnosis of mental disorders. For instance, it does not account for the high degree of co-occurrence of mental disorders with each other, nor does it account for the relative importance of various symptoms or for the significant proportion of patients suffering from subthreshold diagnosis. This growing awareness has motivated recent research on dimensional diagnostic systems that span multiple units of analysis from behaviour to neurobiology (see e.g., Research Domain Criteria "RDoC").

The overall challenge is to come up with effective and cost-efficient solutions for diagnosis and treatment of mental disorders even at a subthreshold stage that can actually be deployed at large scale.

Applying cutting-edge technology using multimodal measurement systems holds the promise to provide better transdiagnostic behavioural and neural measures of cognitive and affective disruption across psychotic disorders.

Neuroscience knowledge and technology is still largely restricted to the realm of academia and large medical centers. This is due to a combination of factors including:
- 1) expensive equipment and procedures involving highly trained experts (e.g., MRI);
- 2) limitations of experimental paradigms, which most often are restricted to simple artificial stimulations; and
- 3) lack of commercial solutions exploiting new technologies.

It has been proposed to diagnose cognitive disorders using quantitative electroencephalography (EEG), for example in Kanda et al, 2009, "The clinical use of quantitative EEG in cognitive disorders", Dementia & Neuropsychologia , 3(3), 195-203 and in Kesebir et al (2018). "QEEG in affective disorder: about to be a biomarker, endophenotype and predictor of treatment response". Heliyon, 4 (8), e00741. But these publications only discuss the use of quantitative EEG as a biomarker and not its implementation in a real solution in a concrete tool. Moreover, the use of EEG alone as a biomarker is tricky as EEG signals are a combination of both neuronal signals and artefacts (ocular, motion...) and therefore, interpretations of EEG markers are highly dependent on the experimental protocol: stimulation used (nature, type...), control conditions (to contrast and remove noise), subject position... Without a very balanced design (simple design to have tight control of subject exposition, inclusion of control conditions, strength of the measured effects in terms of subject reactivity...) the EEG markers are weak and cannot be used in a commercial solution.

To address the drawbacks mentioned before, there is a need for a solution that diagnoses mental disorders using neuromarkers and that is faster than the state of the art, cost effective and reliable.

### SUMMARY OF THE INVENTION

The present invention solves the above-mentioned problems by providing a solution able to detect automatically psychological traits and preferably mental disorders even at pre-clinical stages.

According to a first aspect of the invention, this is satisfied by providing a method carried out by a computer for detecting psychological traits of a user and preferably mental disorders, the method comprising at least the steps of:
- Providing to the user a predetermined sequence of virtual environments, each virtual environment of the sequence of virtual environments being related to a predetermined valence and having a predetermined duration,
- Providing, during the sequence of virtual environments, a passive oddball paradigm to the user comprising providing standard sensory stimulation for a predetermined duration and deviant sensory stimulation for a predetermined duration,
- Acquiring, during the sequence of virtual environments, at least one electroencephalography signal,
- Computing an Event-Related Potential waveform, for each predetermined valence and for each standard and deviant sensory simulation of the oddball paradigm, based on the electroencephalography signal,
- For each valence, computing mismatch negativity based on a difference between the computed Event Related Potentials during deviant sensory stimulation and Event Related Potentials during standard sensory stimulation,
- For each valence, computing a peak around 300 ms post trigger based on the difference between the computed Event Related Potentials during deviant sensory stimulation and Event Related Potentials during standard sensory stimulation,
- Detecting a psychological trait based on the computed mismatch negativity for each valence, on the peak around 300ms for each valence and on predetermined thresholds.

In the invention, quantitative electrophysiology is applied to assess cognitive and affective disruption across psychotic disorders, with a special focus on determining early biomarkers of chronic stress, anxiety and depression. Indeed, in the invention, electroencephalography is used and analysed to correctly assess psychological traits and preferably mental disorders of a user, thus providing an automatic, fast and reliable detection method.

The invention permits to carry out computer-based psychological traits detection thanks to a predetermined sequence of virtual reality environment to create emotional and cognitive relevant situations, enabling to dive the user in a particular scenario or environment to evaluate its reaction. Virtual Reality technology is used to immerse participants in situations with varying levels of emotional demand, thereby inducing mental and physiological states of interest in the context of the identification of cognitive and emotional disruptions, such as tolerance to high or very low cognitive load, attention bias and capture, affective reactivity and resilience.

The reaction of the user is then evaluated based on a computed Event-Related Potential from an electroencephalogram, permitting an objective evaluation. An oddball paradigm is used to evoke said Event-Related Potential. The attentional focus of the subject in the Virtual Reality scene is measured objectively from the Event-Related Potentials response to the oddball task. This implies a dual task that the subject is exposed to, namely, the immersion in a Virtual Reality emotional scene and a passive auditory oddball. The brain of the user tackles both these tasks simultaneously. The oddball Event-Related Potentials measures give a way to quantify objectively the impact of the Virtual Reality scene on the participant: the stronger it is, the weaker the Event-Related Potentials amplitude in response to the oddball will be. Indeed, human attentional capacities are limited, therefore, if the user is focused on one task (here, the Virtual Reality immersion), then the user's neuronal responses to a second, even passive, task will be lowered. By choosing adequately the Virtual Reality scenes, it is thus possible to quantitatively measure the scene impact on the user and to link this impact to psychological aspects such as subclinical depression, stress or anxiety, as will be shown later in the description.

The invention is not limited to the detection of mental disorders but can also be applied to the study of the cerebral responses to attentional immersion in a field of applications ranging from advertising tests to visual messages for driving vehicles (cars, airplanes) which will be encompassed in psychological traits. It is understood by "psychological trait" ....

The method according to the invention may also have one or more of the following characteristics, considered individually or according to any technically possible combinations thereof:
- each virtual environment of the sequence of virtual environments is related to a positive valence, to a negative valence or to a neutral valence,
- the detected mental disorder is depression, anxiety or stress,
- the virtual environments of the sequence of virtual environments are 360-degree videos,
- in the step of providing passive oddball paradigm, the provided sensory stimulations are auditory, visual or tactile stimulations,
- the provided sensory stimulations are auditory or visual and are provided by the virtual reality device.

Another aspect of the invention relates to a system for detecting a psychological trait of a user, the system comprising:
- at least one electroencephalograph configured to carry out the step of acquiring the at least one electroencephalography signal of the method according to any of the preceding claims,
- at least one virtual reality device configured to carry out the step of providing to the user the predetermined sequence of virtual environments,
- at least one processor configured to carry out the computing steps and the step of detecting the psychological trait of the method according to any of the preceding claims.

The virtual reality device may further be configured to carry out the step of providing the passive oddball paradigm to the user of the method according to the invention.

Another aspect of the invention relates to a computer program product comprising instructions which, when the program is executed by a computer, causes the computer to carry out the method according to the invention.

Another aspect of the invention relates to a computer-readable medium comprising instructions which, when executed by a computer, cause the computer to carry out the method according to the invention.

The invention finds a particular interest to automatically detect anxiety, stress and depression disorders at pre-clinical stages in a fast and cost-effective way.

### BRIEF DESCRIPTION OF THE FIGURES

Other characteristics and advantages of the invention will become clear from the description that is given thereof below, by way of indication and in no way limiting, with reference to the appended figures, among which:
- Figure 1 is a schematic representation of a system configured to carry out a psychological trait detection method according to the invention,
- Figure 2 is a schematic representation of a psychological trait detection method according to the invention,
- Figure 3 shows a sequence of virtual environments of the psychological trait detection method according to the invention,
- Figure 4 shows event-related potential waveforms for standard and deviant stimulations according to the invention,
- Figure 5 shows the mismatch negativity in neutral valence as a function of the stai score,
- Figure 6 shows the difference of mismatch negativity between negative and neutral valences as a function of the stai score.

### DETAILED DESCRIPTION

For greater clarity, identical or similar elements are marked by identical reference signs in all of the figures.

The figures are provided for information purposes only and are in no way limiting the invention to their disclosure.

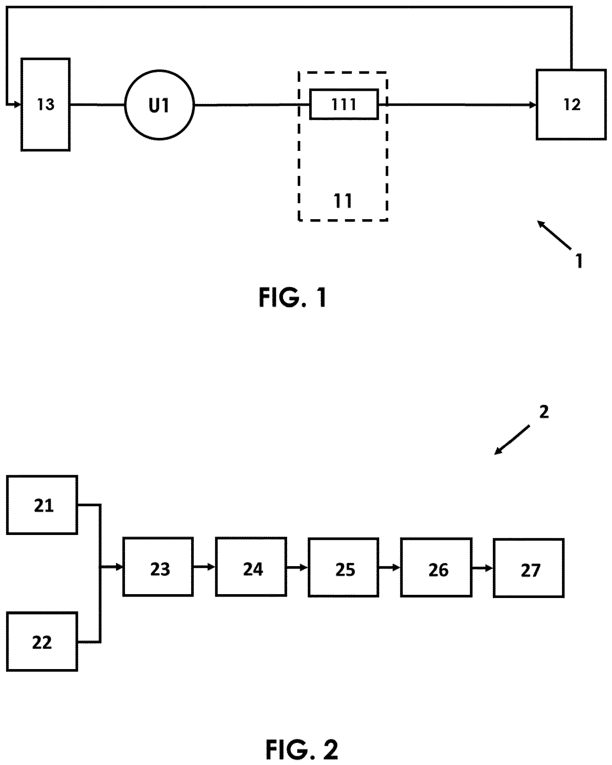

Figure 1 presents the system configured to carry out the group biofeedback method of the invention.

The psychological trait detection system 1 represented at Figure 1 comprises a plurality of biological sensors 11, a processor 12 and a virtual reality device 13. The psychological trait detection system 1 is used by user U1 to detect a psychological trait of user U1, and preferably a mental disorder.

The biological sensors 11 comprise an electroencephalograph 111 configured to acquire an electroencephalogram of the user U1.

The electroencephalograph 111 comprises electrodes placed along the scalp of the user U1 to acquire an electroencephalogram of the user U1. The electroencephalogram comprises signals acquired by each of the electrodes of the electroencephalograph 111.

The electroencephalograph 111 preferably has dry electrodes so as to be easily used by user U1.

The psychological trait detection system 1 further comprises at least one virtual reality device 13. The virtual reality device 13 enables to provide to user U1 different virtual environments. The virtual reality device 13 is preferably a headset, comprising sound and image means, and optionally position means. The system 1 can, in an alternative, comprise several virtual reality devices 13, for example one to provide images and one to provide the sound and optionally one to provide positioning means. The sound means permit to provide stereo sound to the user U1 and are for example headphones. The image means permit to provide stereoscopic images such as stereoscopic videos to the user U1 and are for example screens. The position means are optional, permit to acquire the movements of the user U1 for example to move the user in a virtual environment and are for example accelerometers.

It is understood by "virtual environment" at least a 360-degree image, preferably a 360-degree video, also known as immersive video, or any other virtual environment such as a computer-simulated environment.

The psychological trait detection system 1 also comprises at least one processor 12 configured to carry out some of steps of the method according to the invention. This will be described with the description of Figure 2. When a component of the system 1 is said to be carrying out a step of a method, the component executes instructions stored by a memory (not represented), the instruction causing the component to carry out said step.

The different components of the system 1 exchange information using data transmission channels. These data transmission channels can be implemented using wired means or wireless means. Wireless means include any wireless transmission device or system using wireless exchange protocols including but not limited to Wi-Fi^{®}, Bluetooth^{®}, LoRa^{®}, ZigBee^{®} or any other wireless protocol and wired means include any wired transmission device or system.

Figure 2 shows a schematic representation of a psychological trait detection method according to the invention.

The psychological trait detection method 2 represented at Figure 2 comprises eight steps. Each step of the method is carried out by at least part of the psychological trait detection system 1.

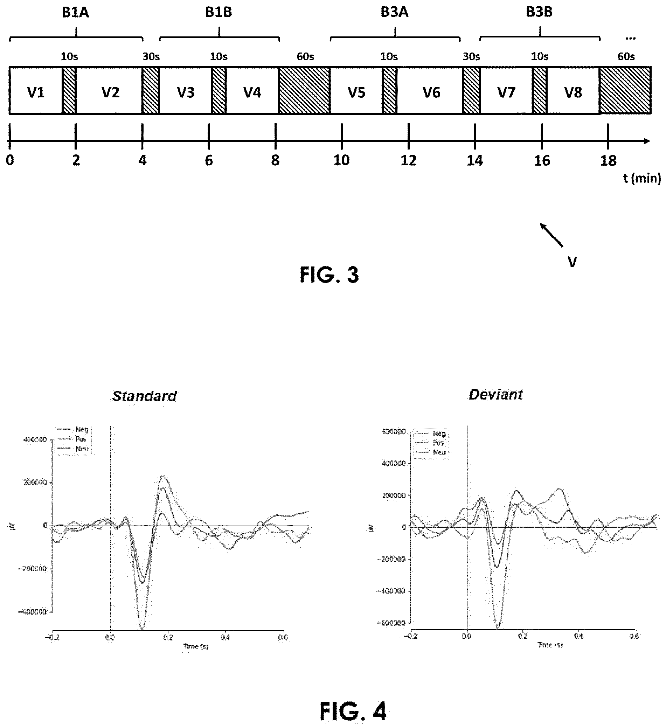

In a first step 21, a predetermined sequence of virtual environments is provided to the user U1. Each virtual environment of the sequence of virtual environments is related to a predetermined valence and has a predetermined duration. An example of a sequence of virtual environments is represented at Figure 3.

Figure shows a schematic representation of a sequence of virtual environments according to the invention.

The predetermined sequence of virtual environments V comprises several virtual environments V1 to V8, separated by no stimulation (baseline periods).

The virtual environments V1 to V8 are each related to a predetermined valence described in the following table:

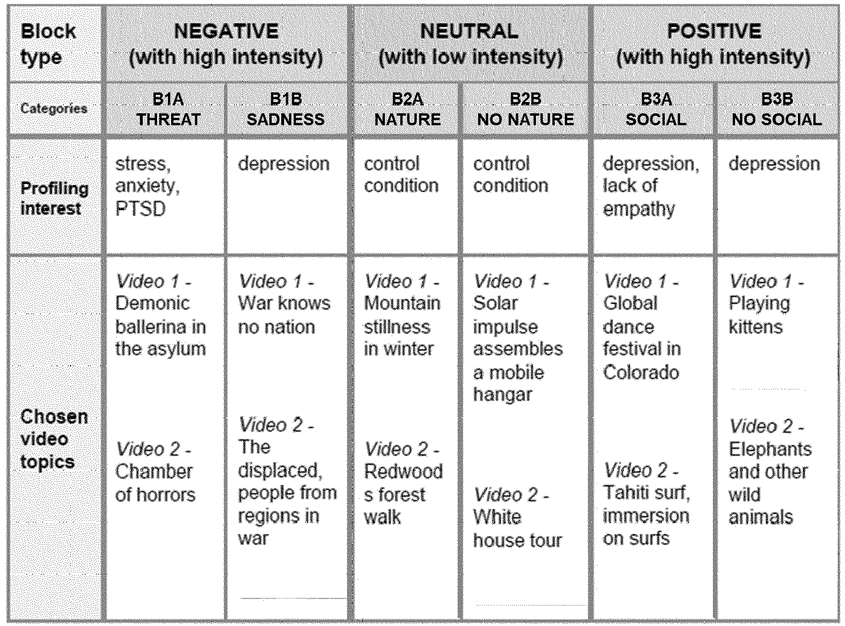

The virtual environments V1 to V8 each belong to a category B1A, B1B, B2A, B2B, B3A or B3B of the table. For example, a virtual environment belonging to category B1A is related to a negative valence, while a virtual environment belonging to category B3B is related to a positive valence. A virtual environment belonging to category B2B is related to a neutral valence. Valence permits to evaluate the emotional charge of a virtual environment, as the term valence in psychology is used to refer to the intrinsically pleasant or unpleasant quality of a stimulus or situation.

For example, a virtual environment related to a negative valence can be a virtual environment diving user U1 in a horror environment. This permits to evaluate the user U1's response to threat (see category B1A in the table) to later evaluate if the user U1 has stress, anxiety or PTSD (Post-Traumatic Stress Disorder). Indeed, it is known that in anxiety, there are higher impacts of negative emotions and, more specifically, there is attentional bias to threatening materials, as shown in Goodwin H. et al (2017). "Generalized Anxiety Disorder, worry and attention to threat: A systematic review." Clinical Psychology Review, 54, 107-122*.* A virtual environment related to a positive valence can be a virtual environment diving user U1 in a concert. This permits to evaluate the user U1's response to social events (see category B3A in the table) to later evaluate if the user U1 has depression or lack of empathy. Indeed, in depression, positive emotional contents are rated less positive than in healthy subjects.

Providing a sequence of virtual environments related to different valences enables to evaluate the reactions of user U1 in the different environments to have a more accurate and objective evaluation of psychological trait. This is not possible in the state of the art, where the environment of the evaluation cannot be changed.

The sequence of virtual environments represented at Figure 3 is only an example and different sequences can be used. In the example, the virtual environments are immersive videos, i.e. 360-degree videos. In the example, two virtual environments are chosen for each valence and are displayed one after the other. This enables to control for possible affective relation to specific environments, for instance for positive immersion, a musician could have specific reaction to a concert and more classical reaction to kittens, therefore, it levels those confounds. The sequence comprises baseline periods of different durations. To compute Event Related Components, baseline recordings are used to correct, therefore, baseline periods of 10 seconds are included between each video of the same category. Baseline periods of 30 seconds are included between each category of the same emotional block. Baseline periods of 60 seconds are included between each emotional block. The design of the sequence by "blocks" to ensure that the emotional arousal is coherent inside a block (to carry on effects of emotional reactions). Furthermore, when used by several users, the virtual environment blocks can be randomized.

The virtual environments can be stored in a memory (not represented) and provided to the user U1 by the virtual reality device 13 following instructions from the processor 12. The virtual environments can also be provided by the virtual reality device 13 to user U1 in any other way, i.e. following instructions from any other device or system. The order of the virtual environments of the sequence of virtual environments can be predetermined randomly or predetermined based on entered or retrieved data, as long as the virtual environment presented to user U1 are separated by baseline slots.

In a second step 22, simultaneous with the step 21 of providing the sequence of virtual environments, an oddball task is provided to user 21. This can be done using the virtual reality device 13 or using an ad hoc device. The oddball task is preferably an auditive oddball. Preferably again, the oddball task is passive, meaning that the user U1 does not have a physical task to accomplish and that the subject detects automatically and even non consciously the deviant signals. The passive oddball task consists in hearing passively two types of beep tones: a standard beep tone and a deviant beep tone. The standard beep tone is frequent and the deviant beep tone is rare which induces a specific neuronal response of the brain which switches its attention to the rare event. Therefore, by contrasting these two types of events, it is possible to compute specific electroencephalography Evoked Potentials ("EP", or ERPs for Event Related Potentials) which are markers of the deviant detection by the brain. The computation of EEG ERP will be described later.

Passive oddball paradigm has been studied extensively and is used in clinical EEG studies including: schizophrenia *[*Alustiza I. et al. (2018). "Aberrant timing and oddball detection in Schizophrenia: findings from a signed differential mapping meta-analysis." Heliyon, 4 (12), e01004*.];* attention deficit/ hyperactivity disorder *[*Janssen, T. W. P. et al. (2016). "An ERP source imaging study of the oddball task in children with attention deficit/hyperactivity disorder. " Clinical Neurophysiology, 127 (2), 1351-1357*.],* autism *[*Sokhadze, E. et al. (2017). "Atypical Processing of Novel Distracters in a Visual Oddball Task in Autism Spectrum Disorder." Behavioral Sciences , 7 (4), 79*.]* and in emotional disorders (depression, posttraumatic stress disorder, maltreatment, social anxiety) [Schlüter, H., & Bermeitinger, C. (2017). "Emotional Oddball: A Review on Variants, Results, and Mechanisms." Review of General Psychology, 21 (3), 179-222*.].* It has also been used to discriminate between job involvement [Uther, M. et al (2018). "Email Overload? Brain and Behavioral Responses to Common Messaging Alerts Are Heightened for Email Alerts and Are Associated With Job Involvement." Frontiers in psychology, 9], emotional sensitivity [Sanger, 2017], motherly attachment, psychopathy tendencies, job burnout, sleep deprivation [Schlüter, H., & Bermeitinger, C. (2017). Emotional Oddball: A Review on Variants, Results, and Mechanisms. Review of General Psychology, 21 (3), 179-222*.].* The combination with virtual reality and the rest of the system and method permits to control the environment of the user and thus to automatically and reliably detect psychological trait and preferably mental disorders.

For example, when the oddball task is an auditory passive oddball task, the following parameters can be used: The oddball task comprises two types of tones lasting 100 ms: one standard tone (75% of the trials) with a pitch at 750 Hz and a deviant tone (25% of the stimuli) with a pitch at 1000 Hz. The inter stimulus interval varied randomly from 1000 to 1300 ms. The auditory level can be controlled with a sonometer to be of 75 dB. To ensure that the participants can correctly hear the stimuli, the virtual environments soundtracks can be kept low and can omit the target tone frequencies (750 and 1000 Hz).

The reaction of the body and the brain to the virtual reality environments are registered by a combination of different physiological sensors to quantitatively assess the individual cognitive and affective profile. The specific design of this evaluation paradigm allied to the complementary of the signals records an accurate quantification of the cognitive and emotional functioning of the participant during virtual reality exposure. The attentional engagement in the virtual reality environments is quantified by the EEG (electroencephalography) response to the oddball stimulation.

In a step 23, at least one electroencephalography signal of the user U1 is acquired. The EEG is acquired during the sequence of virtual environments so as to reflect the reactions and the attentional engagement of the user U1 during the virtual reality stimulation in the virtual reality environments. The EEG is acquired using the electroencephalograph 111 presented previously. An example implementation of signal processing of the EEG signal will now be described. First, the acquired signals are band-passed filtered between 1 and 16 Hz using a 4th order butterworth iir (infinite impulse response) filter and resampled at 128 Hz. Then, the remaining files are converted into an open-source Python package for exploring, visualizing, and analysing human neurophysiological data called "MNE", a standard montage is applied to position the 12 electrodes of the electroencephalograph 111. To remove the blinks artefacts, an independent component analysis infomax is run to identify the "bad" components (based on their topographies and time course) and remove them. Then, "bad" channels are identified (based on their spectrum and time course) and interpolated. An average referencing is then applied. Finally, "bad" time windows (with artefacts) are identified and excluded. The signals are epoched into the different valences (neutral, positive and negative for both standard et deviant signals) and an additional artefact detection is run to remove the trials with an amplitude higher than 80 uV. This permits to obtain relevant signals to later computer Event Related Potentials.

In a step 24, an Event-Related Potential waveform is computed for each predetermined valence and for each standard and deviant sensory simulation of the oddball paradigm, based on the electroencephalography signal. The computed Event-Related Potential Waveforms are represented at Figure 4 for standard and deviant stimulations. The computation is carried out by the processor 12 executing instructions stored in a memory. The computation of an Event-Related Potential for each valence permits to evaluate the attentional engagement of user U1 objectively for different valences thanks to different virtual reality stimulations. The Event Related Potentials waveforms were computed on EEG signals to which baseline correction was applied, where the baseline can be defined as 150-0 ms before the triggers. Event Related Potentials (ERPs) are computed on channel selection, covering the fronto-central regions, for example based on electrodes FC3, FCz, FC4, Cz, C3 and C4.

The step 25 comprises, for each valence, computing mismatch negativity ("MMN") based on a difference between the computed Event Related Potentials during deviant sensory stimulation and Event Related Potentials during standard sensory stimulation. The mismatch negativity is a known component of the Event Related Potential. It represents the discordance between ERP waveforms in standard and deviant oddball stimulations. The auditory mismatch negativity is a negative potential in the fronto-central regions, with a typical latency around 150-250 ms after the trigger of the deviant stimulus. It is understood by "computing a mismatch negativity" the automated retrieval by computer of the amplitude of a peak around 150-250ms of the waveform of the difference between the computed Event Related Potentials during deviant sensory stimulation and Event Related Potentials during standard sensory stimulation.

The step 26 comprises, for each valence, computing a peak around 300 ms post trigger based on the difference between the computed Event Related Potentials during deviant sensory stimulation and Event Related Potentials during standard sensory stimulation. This step is also carried out by the processor 12 of the system 1. This peak is called the P3a. It is understood by "computing a peak" the automated retrieval by computer of the amplitude of said peak of the waveform of the difference between the computed Event Related Potentials during deviant sensory stimulation and Event Related Potentials during standard sensory stimulation.

Both the P3a and the MMN are linked to the automatic switch in the attentional focus [Escera, C. et al (2003). "Attention capture by auditory significant stimuli: semantic analysis follows attention switching." European Journal of Neuroscience,18 (8), 2408-2412*.]* and to the engagement of the subject in the emotional scene [Barbosa, F. et al (2019). "Attention allocation to 2D and 3D emotion-inducing scenes: A neurophysiological study." Neuroscience letters , 698 , 165-168.]. A major advantage of these components is that they are present even when the users are not motivated by the task, e.g. even during sleep and coma.

In a step 27, the processor 12 detects a psychological trait based on the computed mismatch negativity for each valence, on the peak around 300ms for each valence and on predetermined thresholds for each valence. The predetermined threshold can be predetermined based on measures (i.e. EEG) on healthy users and on users affected by mental disorders, when trying to detect mental disorders. The predetermined threshold can be based on a comparison between negative valence and neutral valence and on a comparison between positive valence and neutral valence, thus using neutral valence measurements as a control condition.

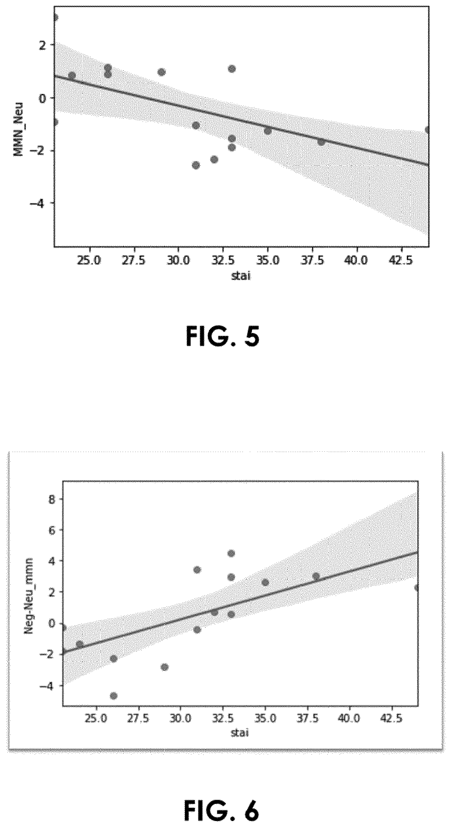

Figure 5 and Figure 6 both show a mismatch negativity computation as a function of STAI (for "State-Trait Anxiety Inventory") scores. STAI scores were obtained based on self-reports using questionnaires.

As represented at Figure 5 showing the mismatch negativity for neutral valences, it is found that the more the subjects are anxious (i.e. the higher the STAI score) the more negative is the MMN amplitude. This first result indicates that the deviant detection in the neutral conditions is stronger in the anxious users, therefore they seem to be less attentive to the neutral environment and more attentive to the surrounding (potentially threatening) information. A high negative amplitude of MMN for neutral valence thus can result in detecting anxiety of user U1 by the processor 12.

As represented in Figure 6 showing the difference in mismatch negativity between negative and neutral valences, it is also found that the MMN for the Negative minus the Neutral condition is correlated with the STAI scores (R = 0.67, p<10-17). This difference of MMN between neutral and negative conditions indicates the diminution of attentional disposition to the oddball in the negative condition compared to the neutral one and therefore it is a measure of the attentional involvement in the negative environment. Therefore, this result indicates that the more the subjects are anxious, the stronger they are captured and attentive to the negative scenes. This also makes sense as a negative scene carries potential risk or threatening information to which the anxious individual raises his attention.

It is also found that STAI scores (measuring anxiety) are correlated with the P3a differences between neutral and negative emotional blocks (R=0.47, p<10-17), which is congruent with the previous result.

Furthermore, the participant's brain response to the oddball in negative and neutral environments is sufficient to detect anxious subjects.

## Claims

1. Method carried out by a computer for detecting a psychological trait of a user, the method comprising at least the steps of:
- Providing to the user a predetermined sequence of virtual environments, each virtual environment of the sequence of virtual environments being related to a predetermined valence and having a predetermined duration,
- Providing, during the sequence of virtual environments, a passive oddball paradigm to the user comprising providing standard sensory stimulation for a predetermined duration and deviant sensory stimulation for a predetermined duration,
- Acquiring, during the sequence of virtual environments, at least one electroencephalography signal,
- Computing an Event-Related Potential waveform, for each predetermined valence and for each standard and deviant sensory simulation of the oddball paradigm, based on the electroencephalography signal,
- For each valence, computing mismatch negativity based on a difference between the computed Event Related Potentials during deviant sensory stimulation and Event Related Potentials during standard sensory stimulation,
- For each valence, computing a peak around 300 ms post trigger based on the difference between the computed Event Related Potentials during deviant sensory stimulation and Event Related Potentials during standard sensory stimulation,
- Detecting a psychological trait based on the computed mismatch negativity for each valence, on the peak around 300ms for each valence and on predetermined thresholds.

2. Method according to the preceding claim **characterized in that** the detected psychological trait is a mental disorder.

3. Method according to any of the preceding claims **characterized in that** each virtual environment of the sequence of virtual environments is related to a positive valence, to a negative valence or to a neutral valence.

4. Method according to claim 2 **characterized in that** the detected mental disorder is depression, anxiety or stress.

5. Method according to any of the preceding claims **characterized in that** the virtual environments of the sequence of virtual environments are 360-degree videos.

6. Method according to any of the preceding claims **characterized in that**, in the step of providing passive oddball paradigm, the provided sensory stimulations are auditory or tactile stimulations.

7. Method according to the preceding claim **characterized in that** the provided sensory stimulations are auditory and are provided by the virtual reality device.

8. System for detecting a psychological trait of a user, the system comprising:
- at least one electroencephalograph configured to carry out the step of acquiring the at least one electroencephalography signal of the method according to any of the preceding claims,
- at least one virtual reality device configured to carry out the step of providing to the user the predetermined sequence of virtual environments,
- at least one processor configured to carry out the computing steps and the step of detecting the psychological trait of the method according to any of the preceding claims.

9. System according to claim 8, **characterized in that** the virtual reality device is further configured to carry out the step of providing the passive oddball paradigm to the user of the method according to any of claims 1 to 7.

10. Computer program product comprising instructions which, when the program is executed by a computer, causes the computer to carry out the method according to any one of claims 1 to 7.

11. Computer-readable medium comprising instructions which, when executed by a computer, cause the computer to carry out the method according to any one of claims 1 to 7.
